# EUROPEAN PATENT SPECIFICATION

(11) **EP 0 958 864 B1**
(45) Date of publication and mention of the grant of the patent: **13.10.2004**
(21) Application number: 99110099.1
(22) Date of filing: 25.05.1999
(51) Int. Cl.: B05B 9/04, B05B 12/02, A01M 13/00, A61L 2/22, A61L 9/14, B65F 7/00

(54) **Process for desinfecting and/or deodorising compartments, a device and compartment for carrying out the process**
Verfahren zum Desinfizieren und zur Deodorierung von Abteilungen, Vorrichtung und Abteilung zum Ausführen des Verfahrens
Procédé pour désinfecter et/ou désodoriser des compartiments, dispositif et compartiment pour la mise en oeuvre du procédé

(30) Priority: 22.05.1998 IT PR980034; 07.07.1998 IT PR980042
(43) Date of publication of application: 24.11.1999
(73) Proprietor: Schia, Lorenzo, 43048 S. Andrea Bagni (PR) (IT)
(72) Inventor: Schia, Lorenzo, 43048 S. Andrea Bagni (PR) (IT)
(74) Representative: Guareschi, Antonella

(56) References cited:
- EP-A- 0 137 416
- EP-A- 0 216 734
- EP-A- 0 816 259
- WO-A-00/50320
- WO-A-95/01295
- GB-A- 399 031
- GB-A- 2 251 781
- US-A- 4 211 745
- US-A- 4 242 311
- US-A- 4 694 846

## Description

This Invention relates to a process for disinfecting and/or deodorising compartments in need of disinfection and/or deodorisation, especially if in contact with waste material, such as by a disinfecting liquid nebulised from a disinfection tank via a nozzle, and a device for carrying out the process. The invention relates also to a compartment, in which the process can be carried out, as well as to a waste bin in need of deodorisation.

Handling waste material, such as municipal or hospital refuse, is a hard job. Therefore, it is frequently done by a staff of foreigners educated under different civilatory conditions. Nevertheless, handling has to be done carefully, considering the hygienic requirements. This is especially necessary in a time in which infectious diseases could be propagated by carelessness. It has been found that a device as disclosed in EP-A-0 848 999 is particularly suited to prevent spreading of such diseases. Nevertheless, although a disinfecting liquid acts also as a deodoriser by killing foul smell producing bacteria, it is certainly so that a compartment (in its broadest sense) in contact with waste material has a tendency to emit bad odours, even after being disinfected, an example being waste bins, especially after emptying, or some public toilets where a disinfecting liquid is sprayed automatically after closing the door.

It is known from the U.S. Patent 4.211.745 the spray nozzles spray an insecticide, a deodorant, and a rust preventative lubricant only into the container after it has been emptied, but before it is lowered to the ground.

It is known from the U.K. Patent Application 2.251.781 a waste bin provided with deodorising apparatus for dispensing the deodorising powder only when the lid is moved between an open position and a closed position.

Therefore, it is one object of the present invention to improve the said situation. It is a further object of the invention to solve the task of hygienisation (and/or deodorisation which is not usually done now) of a waste bin immediately after emptying it as well as disinfecting and/or deodorising its outside and/or the area where it is to be placed back. It is yet another object of the invention to provide for an automatic operation of said hygienisation and/or deodorisation.

In a first step, the invention is based on the recognition that disinfecting the location where bad odours develop is not enough. This is partly due to the fact that too hard disinfecting liquids cannot be used in public (or man accessible) places, because it could also do some harm to persons. Moreover, too hard disinfecting agents have mostly also a disagreeable smell. Development of bad odours can also be due to the fact that their precise origin cannot well be determined. Even the place where a waste bin stands for a longer while can emit some undesirable odours.

The aims of the invention are achieved by the features outlined in the characterizing part of the independent claims; further developments and improvements of the invention are characterized in the depending claims.

In one embodiment, the invention consists in at least one second such disinfecting and/or deodorising step being carried out. Subdividing disinfection into at least two parts enables longer or wider action of the disinfecting liquid, thus more than doubling the success. Moreover, it is possible to subdivide the process in such a manner that first disinfection is done (i.e. by the harder" disinfecting agent) and the second step is directed more towards deodorising, thus removing even those smells which originate from the disinfecting liquid itself.

To facilitate work and to enhance hygienic conditions, a device having the features of claim 3 may be provided according to the invention. Although the problem will be described in the following in the context of disposing household and municipal waste material, e.g. in a waste bin, similar problems may arise in the compartment of e.g. an ambulance car which constitutes a wall-defined room - in which bad odours and/or infective areas may occur - and has at least one door as a closure member. Also surgery and other hospital rooms, hospital and prison wards, waiting rooms and the like may sometimes or often be in need of such treatment. The same applies to other cars, coaches and buses as well as trains, underground and other trams, military crafts and tanks, and aircrafts, such as helicopters, but also to ships, boats and yachts which have to be disinfected and/or deodorised at some occasions or even regularly according to their use. A special application is in toilets, especially in public or portable ones. Another application comprises - especially institutional - kitchens and kitchen hoods, air ducts and the like.

Details of the solutions offered by the present invention will become apparent from the following description of embodiments schematically shown in the drawings, in which:
Fig. 1 shows a lateral view of a lorry for transporting municipal or other waste material in a container according to an embodiment of the present invention;
Fig. 2 is a cross-sectional view along the line II-II of Fig. 1 with a waste bin being emptied;
Fig. 3 is a front view of a preferred waste bin according to the invention; and
Fig. 4 is a lateral view of the waste bin according to Fig. 3.

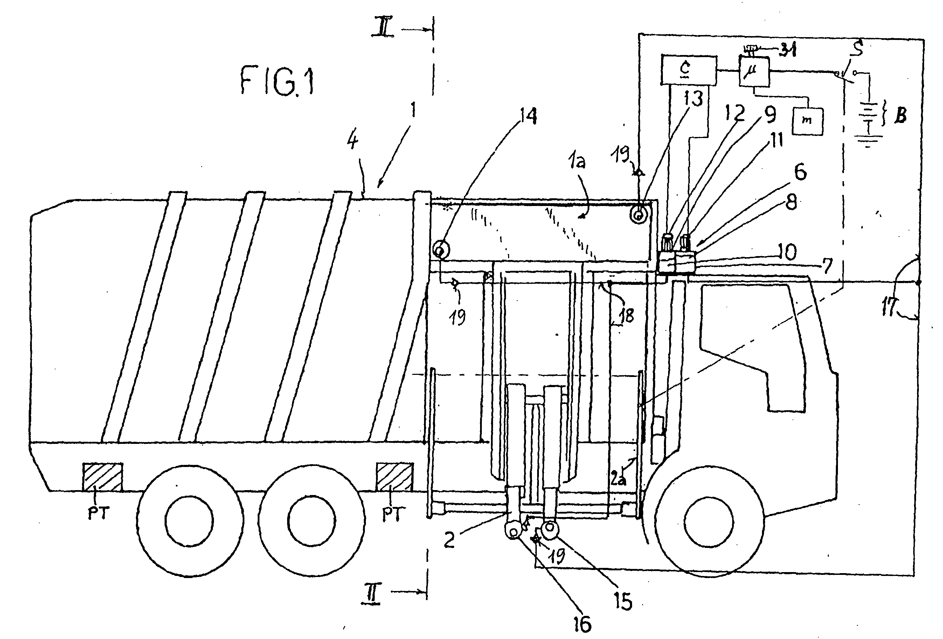

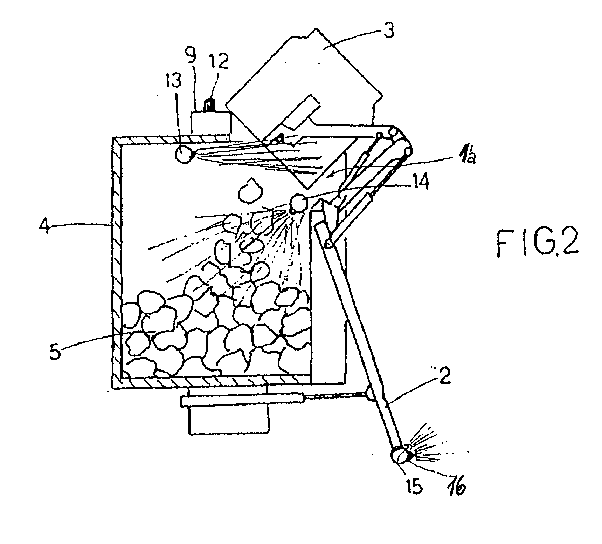

According to Fig. 1, a lorry 1 carries a waste container 4 of any known type. The container has a charging opening 1a through which waste material may be fed. The procedure of feeding waste material 5 into the container 4 is best seen in Fig. 2 where a waste bin 3 is lifted by a known fluidically operated mechanism which needs not be described in detail here. It should only be mentioned that it comprises two lower arms 2.

The lorry 1 is distinguished from any known construction by having a tank enclosure 6 which is subdivided into two different and separate compartments, i.e. a first tank compartment 7 that contains a disinfecting liquid 8, and a second tank compartment 9 which contains a deodorising liquid 10.

A hydraulic pump unit 11 is provided for the tank compartment 7 holding the disinfecting liquid 8 which unit, upon being switched on, delivers the liquid 8 under pressure through a first conduit system 17 towards a first atomising nozzle 13 in the interior of the container 4 as well as towards a second atomising nozzle 15 arranged outside the container 4.

A hydraulic pump unit 12 is provided for the tank compartment 9 holding the deodorising liquid 10 which unit, upon being switched on, delivers the liquid 10 under pressure through a second conduit system 18 towards a first atomising nozzle 14 in the interior of the container 4 as well as towards a second atomising nozzle 16 arranged outside the container 4.

Instead of on the roof of the lorry's cabin, as shown in Fig. 1, alternatively the pump units 11 and 12, together with the respective tanks 7 and 9, may be positioned underneath the chassis, as indicated by the reference sign PT in Fig. 1, or on the other side (not shown) of the lorry.

It will be understood that the present invention is not restricted to the above arrangement, although separation of the conduit systems 17, 18 is preferred. For a waste bin, under certain circumstances only deodorisation may be desirable. If, however, as it is preferred, both disinfection and deodorisation are wanted, it is conceivable to have a common conduit system in which pressure is generated, if desired, even by a single pump, while distribution of the liquids is controlled by valves. Furthermore, it may be suitable to provide non-return valves 19 in the conduits, although this will, in general, not be necessary if the two systems 17, 18 are strictly separated one from another. The pump units 11 and 12 may be of any type and may be replaced by any device capable of generating pressure, such as an injector, a bellow-like arrangement etc. Instead of having a single nozzle 13 or 14 inside and 15 or 16 outside assigned to a respective conduit system 17 or 18, a plurality of nozzles could be used, although a single one for each application has been found to be sufficient.

According to one embodiment of the invention, it has been found to be advantageous if the arrangement shown in Figs. 1 and 2 works during two distinguished and subsequent phases. These phases could, in principle, be carried out manually by operating corresponding manual switches. It is, however, preferable to automate the succession of these phases by using a programmed control, as will be apparent from the following description of one cycle of operation.

In a first phase, the waste bin 3 is unloaded and emptied, as shown in Fig. 2. Solid waste 5 falls into the interior of the waste container 4. To this end, one part 2a of the mechanism for lifting the waste bin 3 (compare Figs. 1 and 2) may be mechanically connected to an initiating switch S to start a program. The program is stored in a memory m and can be read by a micro-processor µ, which is connected to a source of energy, such as a rechargeable battery B, through the switch S.

Initiating the program of memory m results in energising the pump 11 through a control stage C. Thus, the pump 11 draws disinfecting liquid 8 from the tank compartment 7 and delivers it, through the conduit 17, e.g. by a flexible pipe or hose, to the internal nebulizing nozzle 13 and to the external atomising nozzle 15. Thus, as shown in Fig. 2, the interior of the waste bin 3 is disinfected by the nozzle 13, while the nozzle 15 sprays the disinfecting liquid 8 to an area where said waste bin 3 is to be placed and/or to the exterior of the walls of the bin 3, as shown in Fig. 2. Thus, the interior of the waste bin 3, which is in repeated contact with people disposing refuse, is thoroughly disinfected, while, at the same time, either the area where said waste bin 3 is to be (or was) placed and/or the exterior of the walls of the bin 3 are disinfected too so that any risk of an infection is prevented.

Certainly, the disinfecting liquid will also prevent development of odours, because the respective bacteria are killed. Nevertheless, it is useful to deodorise the sites where such odours can already have developed. To this end, a second stage of the process according to the invention is initiated.

This second stage follows immediately the first stage and comprises energising the pump 12 of the tank 9 to supply deodorising liquid 10. The deodorising liquid may suitably be fed through the separate conduit system 18 towards nozzles 14 and 16. Thus, the interior of the waste container 4 is deodorised so that the lorry operators are less bothered by odours, while at the same time the whole area around the waste bin 3 is deodorised which prevents formation of evil smells that may disturb by-passers.

In an alternative procedure, external and internal spraying is done separately in two consecutive steps, although simultaneous spraying externally and internally is preferred.

It is clear that the above two phases could be time-controlled so that they have a shorter or longer duration according to the requirements. Therefore, the circuit may be provided with at least one adjustment device, such as a knob 31, to adjust either the time of the first phase or of the second one or of both.

As shown in Figs. 1 and 2, the external nozzles 15 and 16 may be located on the mechanical arms 2 which uplift and reposition the waste bin 3. Alternatively they may be located on separate arms which may be oriented in a desired direction, i.e. either more in upward direction, as indicated in Fig. 2 (see nozzle 15) or more downwardly (see nozzle 16).

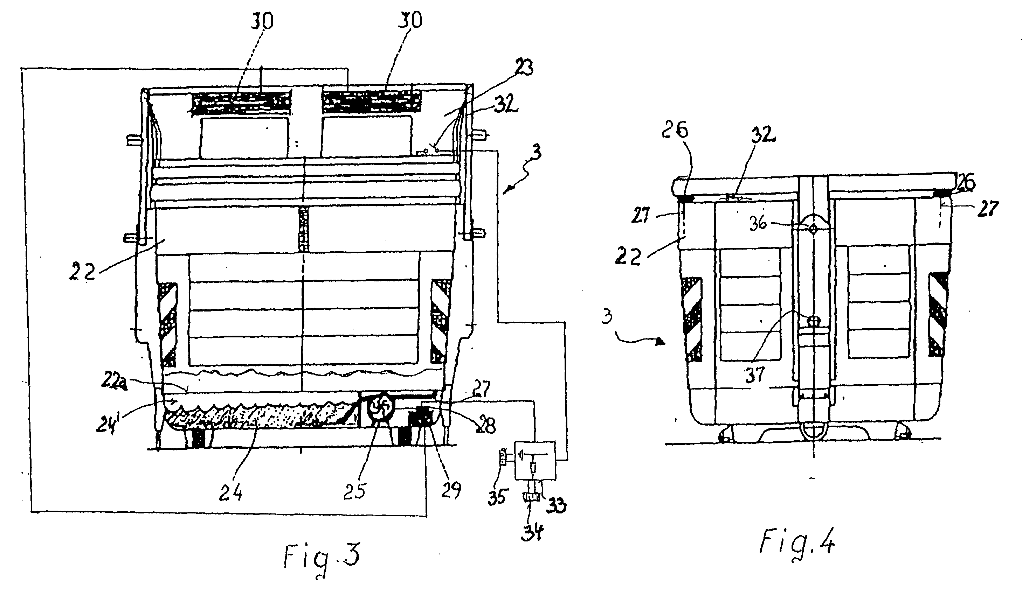

In addition or alternatively, the waste bin 3 may be constructed in the way shown in Figs. 3 and 4. According to this embodiment it is possible to remove odours from the waste bin 3 which may either be done by absorbing them and/or by disinfecting or deodorising its interior. Thus, absorbing could be done by having a lateral and/or bottom compartment containing active coal or another adsorbent.

According to Fig. 3, the waste bin comprises a receptacle or container compartment 22 to be closed by a cover 23. The cover 23 is pivoting about two axes 36, 37, as known per se, to move it from an open position, shown in Fig. 3, into a closed position, shown in Fig. 4. Below the container compartment 22 is a partition wall 22a separating a tank space 24' for an odour removal liquid 24. If a solid absorbent is used instead of the liquid 24, the partition wall 22a could be perforated.

A pump 25 is communicating with the tank space 24' and its liquid 24, as shown. The pump 25 is controlled and energised by an electric control circuit 28 powered by a rechargeable battery 29. In addition or as an alternative, the cover 23 may be provided with solar panels 30 to provide electric energy, either when opened, as in the embodiment shown, or even in its closed position, provided that the panels 30 are outside the waste bin 3.

The pump 25 is able to set the liquid from the tank 24' under pressure and to supply it through a conduit 27 (or a plurality of them) to at least one, preferably a plurality of, spraying nozzle(s) 26 in the top region of the waste bin 3, preferably at its edge. If desired, an opening may be provided in the bottom region of the partition wall 22a to drain liquid sprayed by the nozzles 26, preferably directed inwards, but optionally directed in part to the external walls. Alternatively, external nozzles could be also provided. Instead of having a draining opening close to the partition wall 22a, it would also be possible to have the container compartment 22 closed so as to empty the liquid with emptying the waste material contained in it.

As before, the pump may be replaced by any other equipment able to generate fluid pressure. Furthermore, the tank compartment 24' may be located in any wall of the bin 3 desired. It may even be accommodated within the cover 23 either externally or internally. Moreover, the tank may be subdivided (as in the case of the tank compartments 7, 9 of Fig. 1) to house two different liquids, such as a deodorising liquid and a disinfecting liquid, to be distributed either by two separate conduit systems or by a single, common one.

As shown, the solar panels 30 feed their energy to the rechargeable battery 29 in a manner known per se. Certainly, the panels 30 can also be separated from the bin 3, but in electric contact with the battery 29. Alternatively, the bin 3 has a connection plug to recharge the battery 29 or supply the necessary energy from an external network.

Preferably, a sensor switch 32 (or a position sensor of any type) is provided to sense the position of the cover 23. For in open position of the cover 23 (Fig. 3), it is mostly not desirable to have the nozzles 26 operating. Therefore, when a sensor switch 32 is open (Fig. 3), an electronic circuit 28 controlling the pump 25 is interrupted. However, in the closed position of the switch 32 (Fig. 4), at least one timer unit 33 is activated which causes the pump 25 to be energised in predetermined intervals and/or for predetermined periods, preferably adjusted by an adjusting knob 34 for the intervals and another one 35 for the periods.

From the above description, it will be clear that the whole arrangement, comprising the tanks and pump units, the conduits and non-return valves, the sensor and timer means, the electric lines and batteries etc. could be manufactured as a separate kit to be installed either at the inside or outside of any compartment which according to the invention is in need of hygienisation and/or deodorisation, such as on a container 4 of the lorry 1 or the container 3 according to Figs. 3 and 4, thus forming a disinfecting and/or deodorising apparatus for its own.

It is within the scope of the present invention that numerous modifications could be made; for example two separate tanks 7, 9 could be used instead of having separate compartments of a common tank enclosure 6 which is, nevertheless, preferred. In a simplified embodiment, only the internal nozzles 13, 14) or at least one of them could be used, although using only the external nozzles 15, 16 would also be possible. The nozzles 13 to 16 could be displaceable so as to perform external and internal disinfection and/or deodorisation by a single nozzle or by two separate nozzles.

Furthermore, disinfecting or deodorising the respective compartment may be done in several successive or simultaneous steps, e.g. by disinfecting the interior and/or exterior first, then deodorising them so that the process is carried out in at least four steps.

## Claims

1. Process for disinfecting and/or deodorising a compartment by - in a first step - spraying liquid from a tank holding a disinfecting liquid into the compartment during a predetermined time, and spraying deodorising liquid from a deodorising tank into said compartment, preferably after closing it with a closure member; **characterised in that** simultaneously or consecutively, at least one second step is carried out which is selected from the group consisting of
- spraying disinfecting and/or deodorising liquid to at least part of the outside of said compartment;
- spraying disinfecting and/or deodorising liquid to an area where said compartment is located.

2. Process according to claim 1, for disinfecting a waste bin, whereby the waste bin is unloaded into the container of a lorry, and afterwards optionally closed by moving a cover over its opening, **characterised by** carrying out simultaneously or successively at least two of the following six steps:
a) drawing disinfecting liquid from a disinfecting tank by a pump and supplying it
a3) via the second or a third nebulizing nozzle to an outside area where said waste bin is to be placed;
b) drawing deodorising liquid from a deodorising tank by a pump and supplying it
b2) via the second or a separate nebulizing nozzle to the outside of the waste bin;
b3) via the second or third or a separate nebulizing nozzle to an outside area where said waste bin is to be placed.

3. Device for disinfecting and/or deodorising the waste bin area and the container of the lorry comprising a disinfecting tank (7) communicating with a pump (11) to spray a disinfecting liquid (8) from the disinfecting tank (7) through at least one nebulizing nozzle (13,15) in communication with a conduit (17), **characterised in that** both a disinfecting and a deodorising tank (7;9) - preferably as two separate compartments in a common enclosure (6) - are provided inside or on an installation outside the compartment, and program means (µ, m) are provided for controlling the supply of liquid from either one of said tanks (7, 9) simultaneously or successively, whereby preferably a separate pump (12) and/or at least one separate nebulizing nozzle (14, 16) is/are assigned and communicate exclusively with said deodorising tank (9) through a separate deodorising conduit (18), optionally through at least one non-return valve (19).

4. Device according to claim 3, **characterised in that** it comprises at least one of the following characteristics:
a) timer means (33) for spraying liquid in predetermined intervals and during predetermined periods, wherein preferably at least one of said intervals and said periods are adjustable by adjusting means (34, 35);
b) sensor means (32) for at least one of said positions of said closure member (23), in open position of said closure member (23) feeding said liquid being interrupted.

5. Device according to claim 3 or 4, for disinfecting and/or deodorising a waste bin (3), **characterised in that** said installation outside the waste bin (3) is a wheeled container such as a lorry (1), and said tank (7;9) holding disinfecting and/or deodorising liquid (8;10) respectively is/are in communication with at least one nozzle (13;14) for internal disinfection and/or deodorisation of the waste bin (3), as well as at least one nozzle (15;16) for external disinfection and/or deodorisation of the waste bin (3) and/or of an outside area where the waste bin (3) is to be placed.

6. Device according to claim 5, **characterised in that** one nozzle (14) for internal deodorising is located inside the lorry container (4) and is directed towards the interior of the lorry container (4), while at least one nozzle (15;16) for external disinfection and/or deodorisation is located on an uplifting mechanical arm (2) arranged externally of said lorry container (4), and said nozzles (15;16) being directed towards the support base of said waste bin (3) and/or to said outside area respectively.

7. Waste bin to be periodically deodorised, in that at least one of its walls comprises a space (24') containing solid or liquid deodorising agent (24) which space (24') is separated from the interior compartment (22) of the waste bin (3) by a partition wall (22a) which - in the case of solid deodorising agent (24) - is perforated,
**characterised in that** it comprises at least one of the following characteristics:
a) at least one nebulizing nozzle (26) is located in the upper part of said compartment (22) and/or on said closure member (23);
b) at least one nebulizing nozzle (26) is located outside said compartment for spraying said liquid onto at least one external wall of said compartment (22);
c) timer means (33) for spraying liquid in predetermined intervals and during predetermined periods, wherein preferably at least one of said intervals and said periods are adjustable by adjusting means (34, 35);
d) sensor means (32) for at least one of said positions of said closure member (23), in open position of said closure member (23) feeding said liquid being interrupted;
e) odour removal means (24-35), for example an absorption arrangement;
f) at least one electrically operated device, such as a pump (25);
g) means providing electric current, such as a rechargeable battery (29) and/or a solar energy panel (30).

## Patentansprüche

1. Verfahren zur Desinfizierung und/oder Deodoriserung einer Kammer durch - in einem ersten Schritt - Sprühen von Flüssigkeit aus einem eine desinfizierende Lösung enthaltenden Tank in die Kammer innerhalb einer vorgegebenen Zeit und Sprühen einer deodorisierenden Flüssigkeit aus einem Deodorisierungstank in die Kammer, bevorzugt, nachdem sie mit einem Verschlußelement verschlossen worden ist,
**dadurch gekennzeichnet,**
**daß** gleichzeitig oder aufeinanderfolgend zumindest ein zweiter Schritt ausgeführt wird, welcher aus der Gruppe ausgewählt ist, die aus
- Sprühen einer desinfizierenden und/oder deodorierenden Flüssigkeit auf zumindest ein Teil der Außenfläche der Kammer,
- Sprühen einer desinfizierenden und/oder deodorisierenden Flüssigkeit auf einen Bereich, wo die Kammer angeordnet ist,
besteht.

2. Verfahren nach Anspruch 1 zum Desinfizieren eines Müllbehälters, wobei der Müllbehälter in den Container eines Lastkraftwagens geleert wird und anschließend optional geschlossen werden kann, indem ein Deckel über seine Öffnung bewegt wird,
**dadurch gekennzeichnet,**
**daß** gleichzeitig oder nacheinander zumindest zwei der folgenden sechs Schritte durchgeführt werden:
a) Absaugen einer desinfizierenden Flüssigkeit aus einem Desinfektionstank mit Hilfe einer Pumpe und Zuführen dieser
a3) über die zweite oder eine dritte Vernebelungsdüse auf den Außenbereich, wo der Müllbehälter anzusetzen ist,
b) Abziehen der deodorisierenden Flüssigkeit aus einem Deodorisierungstank mit Hilfe einer Pumpe und Zuführen dieser
b2) über die zweite oder eine getrennte Vernebelungsdüse auf die Außenseite des Müllbehälters,
b3) über die zweite oder dritte oder eine getrennte Vernebelungsdüse auf den Außenbereich, wo der Müllbehälter anzusetzen ist.

3. Vorrichtung zur Desinfizierung und/oder Deodorisierung des Müllbehälterbereichs und des Containers eines Lastkraftwagens, beinhaltend einen Desinfektionstank (7), der mit einer Pumpe (11) verbunden ist, um eine desinfizierende Flüssigkeit (8) aus dem Desinfektionstank (7) über zumindest eine Vernebelungsdüse (13,15) in Verbindung mit einer Leitung (17) zu versprühen,
**dadurch gekennzeichnet,**
**daß** sowohl ein Desinfektions- als auch ein Deodorisierungstank (7;9) - bevorzugt als zwei getrennte Kammern in einem gemeinsamen Gehäuse (6) - innerhalb eines Aufbaus oder auf einem Aufbau außerhalb der Kammer vorgesehen sind, und Programmelemente (µ,m) vorgesehen sind, um die Zuführung von Flüssigkeit aus einem der beiden Tanks (7,9) gleichzeitig oder aufeinanderfolgend zu steuern, wobei bevorzugt eine getrennte Pumpe (12) und/oder zumindest eine getrennte Vernebelungsdüse (14,16) eingesetzt wird/werden und ausschließlich mit dem Deodorisierungstank (9) über eine getrennte Deodorisierungsleitung (18), optional über zumindest ein Rückschlagventil (19) in Verbindung stehen.

4. Vorrichtung nach Anspruch 3,
**dadurch gekennzeichnet,**
**daß** sie zumindest eines der folgenden Merkmale aufweist;
a) Zeitgebereinrichtungen (33) zum Versprühen der Flüssigkeit in vorgegebenen Intervallen und während vorgegebener Perioden, wobei bevorzugt zumindest eines der Intervalle und eine der Perioden durch Justiereinrichtungen (34,35) eingestellt werden können,
b) eine Sensoreinrichtung (32) für zumindest eine der Positionen des Verschlußelementes (23), so daß in der offenen Position des Verschlußelementes (23) die Zuführung der Flüssigkeit unterbrochen wird.

5. Vorrichtung nach Anspruch 3 oder 4 zur Desinfizierung und/oder Deodorisierung eines Müllbehälter (3)
**dadurch gekennzeichnet,**
**daß** es sich bei dem Aufbau außerhalb des Müllbehälters (3) um einen mit Rädern versehenen Container, wie einen Lastkraftwagen (1) handelt und der Tank (7,9), der die desinfizierende und/oder deodorisierende Flüssigkeit (8,10) enthält in Verbindung mit zumindest einer Düse (13,14) steht, um den Müllbehälter (3) innen zu desinfizieren und/oder zu deodorisieren, als auch mit zumindest einer Düse (15,16) um den Müllbehälter (3) außen und/oder einen Außenbereich, an den der Müllbehälter (3) plaziert wird, zu desinfizieren und oder deodorisieren.

6. Vorrichtung nach Anspruch 5,
**dadurch gekennzeichnet,**
**daß** eine Düse (14) für die innere Deodoriserung innerhalb des Lastkraftwagencontainers (4) angeordnet ist und auf das Innere des Lastkraftwagencontainers (4) gerichtet ist, während zumindest eine Düse (15,16) für die äußere Desinfektion und/oder Deodorisierung auf einem mechanischen Hubarm (2) angeordnet ist, der außen an dem Lastkraftwagencontainer (4) angeordnet ist, und die Düsen (15,16) auf die Trägerbasis des Müllbehälters (3) und/oder auf den Außenbereich gerichtet sind.

7. Regelmäßig zu deodorisierender Müllbehälter, bei dem zumindest eine Wand einen Raum (24') enthält, der ein festes oder flüssiges deodorierendes Mittel (24). enthält, wobei der Raum (24') von der inneren Kammer (22) des Müllbehälters (3) durch eine Trennwand (22a) getrennt ist, die - im Falle des festen Deodorisierungsmittels (24) - perforiert ist,
**dadurch gekennzeichnet,**
**daß** er zumindest eines der folgenden Merkmale aufweist:
a) zumindest eine Vernebelungsdüse (26), die im oberen Teil der Kammer (22) und/oder auf dem Verschlußelement (23) angeordnet ist,
b) zumindest eine Vernebelungsdüse (26), die außerhalb der Kammer angeordnet ist, um die Flüssigkeit auf zumindest eine Außenwand der Kammer (22) zu sprühen,
c) eine Zeitgebereinrichtung (33) zum Versprühen der Flüssigkeit in vorgegebenen Intervallen und innerhalb vorgegebener Perioden, wobei bevorzugt zumindest eines der Intervalle und der Perioden durch Justiervorrichtungen (34,35) eingestellt werden kann,
d) Sensoreinrichtungen (32) für zumindest eine Position des Verschlußelements (23), so daß in der offenen Position des Verschlußelementes (23) die Zuführung der Flüssigkeit unterbrochen wird,
e) geruchsentfernende Vorrichtungen (24 bis 35), z.B. eine Absorptionsanordnung,
f) zumindest eine elektrisch betätigte Einrichtung, wie eine Pumpe (35),
g) Einrichtungen, die einen elektrischen Strom bereitstellen, wie eine wiederaufladbare Batterie (29) und/oder eine Solarzellenfläche (30)

## Revendications

1. Procédé pour désinfecter et désodoriser un compartiment en - dans une première phase - vaporisant un liquide provenant d'un réservoir contenant un liquide désodorisant dans le compartiment durant un temps prédéterminé et en vaporisant un liquide désodorisant provenant d'un réservoir désodorisant dans ledit compartiment, préférablement après l'avoir fermé avec un élément de fermeture, **caractérisé en ce que**, simultanément ou consécutivement, au moins une seconde phase est déclenchée, choisie parmi les phases suivantes:
- vaporisation de liquide désinfectant et/ou désodorisant sur au moins une partie de l'extérieur dudit compartiment;
- vaporisation d'un liquide désinfectant et/ou désodorisant sur une zone dans laquelle se trouve le compartiment.

2. Procédé selon la revendication 1, pour désinfecter une benne à ordures, dans lequel la benne à ordures est déchargée dans le conteneur d'un camion, et par la suite éventuellement fermée en mouvant un couvercle sur son ouverture, **caractérisé par** le fait de mettre en oeuvre simultanément ou successivement au moins deux des six phases suivantes:
a) prélèvement d'un liquide désinfectant dans un réservoir désinfectant par une pompe et alimentation du liquide au travers d'une seconde ou troisième buse de nébulisation sur une zone externe dans laquelle ladite benne à ordures doit être placée;
b) prélèvement d'un liquide désodorisant dans un réservoir désodorisant par une pompe et son alimentation
- au travers d'une seconde ou séparée buse de nébulisation sur l'extérieur de la benne à ordures;
- au travers d'une troisième ou séparée buse de nébulisation sur une zone externe dans laquelle ladite benne à ordures doit être placée.

3. Dispositif pour désinfecter et/ou désodoriser la zone de la benne à ordures et le conteneur du camion, comprenant un réservoir désinfectant (7) communiquant avec une pompe (11) pour vaporiser un liquide désinfectant (8) provenant du réservoir désinfectant (7) à travers au moins une buse de nébulisation (13, 15) en communication avec un conduit (17), **caractérisé en ce qu'**aussi bien un réservoir désinfectant qu'un réservoir désodorisant (7; 9) - préférablement sous la forme de deux compartiments séparés dans une enceinte commune (6) - sont prévus à l'intérieur ou sur une installation à l'extérieur du compartiment, et des moyens de programmation (µ, m) sont prévus pour le contrôle de l'alimentation de liquide de l'un desdits réservoirs (7, 9) simultanément ou successivement, par quoi préférablement une pompe séparée (12) et/ou au moins une buse de nébulisation (14, 16) séparée est/sont prévue(s) et communique(nt) exclusivement avec ledit réservoir désodorisant (9) au travers d'un conduit désodorisant (18) séparé, éventuellement au travers d'au moins une valve de non-retour (19).

4. Dispositif selon la revendication 3, **caractérisé en ce qu'**il comprend au moins une des caractéristiques suivantes:
a) des moyens temporisateurs (33) pour vaporiser le liquide à intervalles prédéterminés et pendant des périodes prédéterminées, dans lequel préférablement au moins l'un desdits intervalles et l'une desdites périodes est réglable grâce à des moyens de réglage (34, 35) ;
b) des capteurs (32) pour au moins une desdites positions dudit élément de fermeture (23), l'alimentation dudit liquide étant interrompue dans la position ouverte dudit élément de fermeture (23).

5. Dispositif selon la revendication 3 ou 4, pour désinfecter et/ou désodoriser une benne à ordures (3), **caractérisé en ce que** ladite installation à l'extérieur de la benne à ordures (3) est un conteneur sur roues comme un camion (1), et que ledit réservoir (7; 9) contenant le liquide désinfectant et/ou désodorisant (8; 10) est/sont en communication respectivement avec au moins une buse (13; 14) pour une désinfection et/ou désodorisation de la benne à ordures (3), ainsi qu'avec au moins une buse (15; 16) pour une désinfection et/ou désodorisation externe de la benne à ordures (3) et/ou d'une zone externe où la benne à ordures (3) doit être placée.

6. Dispositif selon la revendication 5, **caractérisé en ce qu'**une buse (14) pour une désodorisation interne est disposée à l'intérieur du conteneur (4) du camion et est dirigée vers l'intérieur du conteneur (4) du camion, alors qu'au moins une buse (15; 16) pour une désinfection et/ou désodorisation externe est disposée sur un bras mécanique de soulèvement (2) disposé à l'extérieur dudit conteneur (4) du camion, et lesdites buses (15; 16) étant dirigées vers la base de support de ladite benne à ordures (3) et/ou respectivement sur ladite zone externe.

7. Benne à ordures devant être périodiquement désodorisée, au moins une de ses parois comprenant un espace (24') contenant un agent désodorisant (24) solide ou liquide, lequel espace (24') étant séparé du compartiment interne (22) de la benne à ordures (3) par une paroi de séparation (22a) qui - dans le cas d'un agent désodorisant solide (24)- est perforée, **caractérisée en ce qu'**elle comprend au moins l'une des caractéristiques suivantes:
a) au moins une buse de nébulisation (26) est située dans la partie supérieure dudit compartiment (22) et/ou sur ledit élément de fermeture (23);
b) au moins une buse de nébulisation (26) est située à l'extérieur dudit compartiment de nébulisation dudit liquide sur au moins une paroi externe dudit compartiment (22);
c) des moyens de temporisation (33) pour vaporiser le liquide à intervalles prédéterminés et pendant des périodes prédéterminées, dans lequel préférablement au moins un desdits intervalles et une desdites périodes sont réglables grâce à des moyens de réglage (34; 35);
d) des capteurs (32) pour au moins l'une desdites positions dudit élément de fermeture (23), l'alimentation dudit liquide étant interrompue dans la position ouverte de l'élément de fermeture (23);
e) des moyens d'élimination de l'odeur (24-35), par exemple en une disposition à absorption;
f) au moins un dispositif électrique, comme une pompe (25);
g) des moyens d'alimentation de courant électrique, comme une batterie rechargeable (29) et/ou un panneau à énergie solaire (30).
